# EUROPEAN PATENT SPECIFICATION

(11) **EP 3 561 509 B1**
(45) Date of publication and mention of the grant of the patent: **18.03.2020**
(21) Application number: 18190345.1
(22) Date of filing: 22.08.2018
(51) Int. Cl.: G01N 33/497, A61B 5/097

(54) **PORTABLE DEVICE FOR DETECTION OF BIOMARKERS IN EXHALED AIR AND METHOD OF BIOMARKER DETECTION IN EXHALED AIR**
TRAGBARE VORRICHTUNG ZUM NACHWEIS VON BIOMARKERN IN AUSGEATMETER LUFT UND VERFAHREN ZUR BIOMARKER-DETEKTION IN AUSGEATMETER LUFT
DISPOSITIF PORTABLE POUR LA DÉTECTION DE BIOMARQUEURS DANS L'AIR EXPIRÉ ET PROCÉDÉ DE DÉTECTION DE BIOMARQUEUR DANS L'AIR EXPIRÉ

(30) Priority: 24.04.2018 PL 42531618
(43) Date of publication of application: 30.10.2019
(73) Proprietor: Akademia Gorniczo-Hutnicza im. Stanislawa Staszica w Krakowie, 30-059 Krakow (PL)
(72) Inventor: RYDOSZ, Artur, 31-325 Kraków (PL); MARSZALEK, Konstanty, 31-358 Kraków (PL)
(74) Representative: Bury, Marek

(56) References cited:
- WO-A1-2013/003429
- US-A1- 2007 258 894
- US-B2- 8 922 219
- ANDREAS BERGMANN ET AL: "In Vivo Volatile Organic Compound Signatures of Mycobacterium avium subsp. paratuberculosis", PLOS ONE, vol. 10, no. 4, 27 April 2015 (2015-04-27) , XP055569565, DOI: 10.1371/journal.pone.0123980
- RAMIN GHORBANI ET AL: "Real-time breath gas analysis of CO and CO2 using an EC-QCL", APPLIED PHYSICS B: LASERS AND OPTICS., vol. 123, no. 5, 18 April 2017 (2017-04-18), XP055569567, DE ISSN: 0946-2171, DOI: 10.1007/s00340-017-6715-x
- J KING ET AL: "Isoprene and acetone concentration profiles during exercise on an ergometer", JOURNAL OF BREATH RESEARCH, vol. 3, no. 2, 1 June 2009 (2009-06-01), page 027006, XP055569575, US ISSN: 1752-7155, DOI: 10.1088/1752-7155/3/2/027006
- JULIAN KING ET AL: "A mathematical model for breath gas analysis of volatile organic compounds with special emphasis on acetone", ARXIV.ORG, CORNELL UNIVERSITY LIBRARY, 201 OLIN LIBRARY CORNELL UNIVERSITY ITHACA, NY 14853, 23 March 2010 (2010-03-23), XP080397241, DOI: 10.1007/S00285-010-0398-9
- GROVES W A ET AL: "ANALYZING ORGANIC VAPORS IN EXHALED BREATH USING A SURFACE ACOUSTIC WAVE SENSOR ARRAY WITH PRECONCENTRATION: SELECTION AND CHARACTERIZATION OF THE PRECONCENTRATOR ADSORBENT", ANALYTICA CHIMICA ACTA, ELSEVIER, AMSTERDAM, NL, vol. 371, 1 January 1998 (1998-01-01), pages 131-143, XP001152432, ISSN: 0003-2670, DOI: 10.1016/S0003-2670(98)00294-3

## Description

Present invention is related to a portable device for detection of biomarkers and to a method of biomarker detection in exhaled air. Present invention is related, in particular, to biomarker indicating diabetes.

Current condition of the body, its dysfunctions and some medical conditions may be identified on the basis of presence and concentration of volatile organic compounds, so called biomarkers, in exhaled air. For example, presence of acetone in exhaled air is a diagnostic indication of diabetes.

Polish patent application no. P.423933 discloses a personal, portable device for exhaled air composition monitoring, which includes a housing with an inlet and an outlet for analysed air and a sensor unit system located inside the housing, provided with a system of sensors enabling gas detection and connected with a control module including a sensor signal converter, memory units and a microprocessor. The device is equipped with a first port enabling connection of a mouthpiece pipe, connected to the analysed air inlet leading to the sensor unit via an internal duct and a moisture filter. Such breath-based tests are a promising diagnostic tool which may enable early, non-invasive detection of many diseases-. Their non-invasive nature makes them potentially usable directly by patients using personal devices. Unfortunately, concentration ranges of biomarkers present in exhaled air and providing potential diagnostic indications are often lower than measurement ranges of sensors enabling detection of these compounds, available on the market. Other substances present in the exhaled air, such as water present as vapour and droplet suspension, for example, trammel operation of such sensors. Advanced laboratory techniques, such as mass spectrometry or gas chromatography may not be used in portable and personal devices because of their size, working requirements and costs. The ability to test the concentration of such compounds by patients using portable and personal devices would be highly desirable.

The problem of inadequate sensitivity of semi-conductor sensors was identified in European patent publication no EP2845009B1, which recommends the use of a special reaction mixture and a chemical detection method at home.

An alternative solution to this problem was proposed in the European patent application no. EP2920589, which discloses a sensor with a pre-concentrator - a device used to concentrate gases present in the sample before analysis. Use of this solution enables detection of compounds in exhaled air at concentration lower than the detection threshold of currently used sensors. However, this solution negatively impacts accuracy of detection, for two reasons. Firstly, it leads to an average concentration of detected compounds over the entire breathing cycle and secondly, it requires to include the concentration rate, which varies over time, in the result of the measurement.

Publication no. WO2009025488 of an international patent application discloses a device for analysis of gas composition provided with a control module, a display unit and a set of sensors. Gas fed to the device is dried using a filter.

US2007258894 discloses systems and methods for diagnosing and/or treating diseases as well as monitoring disease treatment. Bodily fluid samples are analyzed using sensor technology to detect the presence of surrogate and/or therapeutic drug markers to provide an efficient and accurate means for diagnosing a disease and/or monitoring disease treatment.

US8922219 discloses a photo ionization detector provided for selectively determining various compounds or gases present in a breath sample. The PID, comprises a substrate comprising a gas ionization chamber, at least one pair of ion sensing electrodes, and at least one amplifying circuit; and an ultraviolet ionization source to transmit a UV light beam into the gas ionization chamber. A system comprises the PID is also provided. A method of detecting a response pattern for various compounds or gases in breath using PID is also provided. Publication of international patent application no WO2013003429 discloses a portable device for detecting biomarkers in exhaled air having a mouthpiece and an air line leading the air from the mouthpiece through a sensor unit to an outlet port. CO₂ sensor connected to the control module is used to trigger measurement.

The objective of the invention is to provide a portable, user-friendly and easy to manufacture device enabling independent measurements of biomarkers in the air exhaled by the patient and a method of detection of these biomarkers.

A portable device for monitoring the composition of exhaled air, provided with a mouthpiece and an air line leading the air from mouthpiece through a sensor unit to an outlet port, wherein the air line includes a moisture filter and the set of sensors is connected with a control unit connected to an internal interface system according to the invention has the air line that also includes a pre-concentrator and a carbon dioxide sensor connected with the control module, and is provided with a module triggering the measurement using a sensor set connected with the pre-concentrator. This configuration allows monitoring of carbon dioxide concentration to be used as a detection method indicating exhalation of the so called "useful air" - the air from pulmonary alveolus. This air is constituted by tidal air and part of expiratory reserve volume which usually remains in the lungs for a longer time and is ejected only if a full exhalation is forced. This air is more saturated with substances produced by the body of the patient - including biomarkers, present therein in higher and easier to detect concentrations, independent from the previous breathing cycle. By detecting high and easy to measure CO2 concentrations we can thus determine the time at which the measurement should be performed in order to analyse useful air and improve working conditions of biomarker sensors, as well as to obtain a more reliable measurement with smaller impact of patient activity. Concentration using the pre-concentrator combined with the use of useful air allows better utilisation of the dynamic range of the sensors. The carbon dioxide sensor is located in the air line upstream of the sensor unit. This allows useful air to be detected in advance and fully utilised. The device is provided with a valve cutting off air supply to the sensor unit located downstream of the carbon dioxide unit. The device is also provided with a second outlet port, while the controlled valve is a three-way valve directing air to the second outlet port in its first position, to the pre-concentrator, the sensor unit and the first outlet port in its second position. Only useful air reaches the pre-concentrator in this configuration, thus ensuring a more precise measurement.

The pre-concentrator and the moisture filter are integrated into a single module including an air duct filled with absorbing and adsorbing compounds. This solution prevents detection sensitivity losses caused by filtration and paradoxically, moisture filters heated cyclically in the pre-concentrator have an extended operational lifetime.

The sensor unit preferably includes an acetone sensor, preferably provided as a semi-conductor sensor. This allows suspected diabetes to be detected using a sensor easily fit inside a portable device.

The method of biomarker detection in exhaled air using a sensor unit, in which an exhaled air stream is fed from the mouthpiece to the outlet using an air line including a moisture filter and a sensor unit according to the invention is characterised in that the exhaled air is concentrated using a pre-concentrator and subjected to a carbon dioxide concentration measurement using a carbon dioxide sensor, while biomarker detection begins when the carbon dioxide indication exceeds a certain threshold. This allows detection in concentrated useful air, namely in conditions most favourable for the sensors.

Before biomarkers are detected using the sensor unit, a calibration measurement is performed using the carbon dioxide sensor, for at least one breathing cycle or even for five breathing cycles and used to determine the threshold carbon dioxide concentration. The threshold concentration is preferably determined as a fraction in the range of 0.8 to 0.95 of the maximum carbon dioxide concentration measured during the calibration measurement.

Preferably threshold concentration is a fraction of 0.91 to 0.93 of the maximum carbon dioxide concentration. In case of significant amount of people within population in this range falls the value of fraction of maximal carbon dioxide concentration above which only useful air reach in biomarkers and relatively free of moisture is exhaled.

Before the biomarker detection, air flow to the sensor unit is preferably cut off using a valve opened again only when the sensor indication is higher than the threshold value. This prevents air other than useful air from reaching the pre-concentrator.

Exhaled air is preferably fed to the second outlet port before the carbon dioxide indication exceeds the threshold value.

The invention is explained below in detail with reference to the attached drawings in which Fig. 1 presents a block diagram of an example embodiment of the invention, Fig. 2 presents an example profile of CO2 concentration changes in exhaled air and a correlated biomarker detection profile, Fig. 3 presents a flow diagram of the method according to the invention, and Fig. 4 presents schematically the pre-concentrator integrated with the moisture filter, intended for use in the disclosed invention.

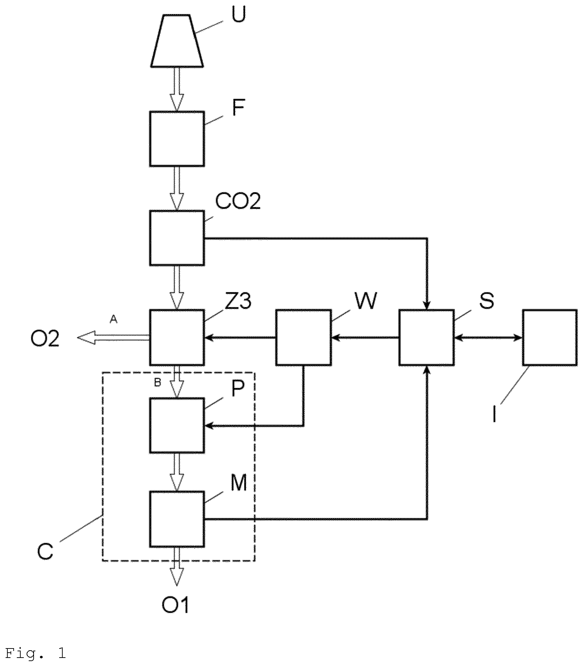

In the first example embodiment of a portable device for monitoring the exhaled air composition, the block diagram of which is presented in Fig. 1, air exhaled by the patient, the line of which has been marked with a double arrow and the flow direction using arrows, passes through the mouthpiece U, the moisture filter F and reaches the carbon dioxide CO2 concentration sensor and the three-way valve Z3 controlled by a signal from the control module S, transmitted using the measurement triggering module W. The three-way valve Z3 in its position A directs the exhaled air to the outlet port O2, while in position B, it directs the air to the measurement compartment C, where the pre-concentrator P and the sensor unit M are located. The air exits the sensor unit M and the compartment C through the outlet port O1.

The moisture filter F is collecting - absorbing - water vapour present in the exhaled air. Vapour presence effectively interferes with gas sensor operation regardless of the detection method: semiconductors, electrochemical or optical methods, thus this interference should be eliminated. The moisture filter may also be provided in one of several forms, e.g. by using silica gel, which changes colour in contact with water vapour and thus signals the need to replace the filter. There are also membrane filters and activated charcoal filters available. Numerous commercial filters of these types are available on the market. Good results were obtained using the Hydro-Therm 1850 filter. The moisture filter is important for the operation of the pre-concentrator, as it prevents absorbing substances from being saturated with moisture from the air. Application of moisture filter allows elimination of the moisture on the result of measurement despite use of pre-concentrator.

The sensor unit M contains at least one sensor. In this particular embodiment this is an acetone sensor.

The pre-concentrator (or microconcentrator) comprises a system performing initial concentration of the exhaled air sample (pre-concentrating system). Its operation should ensure repeatable concentration factor for air samples. At negligible concentration of the tested gas in the sample, it is collected (e.g. Adsorbed) in the microconcetrator until its concentration exceeds the detection threshold of the used sensor, and then suddenly released in response to a control signal. This allows gas detection using a sensor, the detection threshold of which does not allow the gas concentration to be measured directly in the initial mixture (e.g. in mine gases, exhaled air, etc.). A pre-concentration according to the Polish patent publication PAT.225138 is used in this embodiment of the invention. It contains a duct filled with an adsorbent, heated using a heater along a section of its length, while cooling ducts open towards outside are provided along the remaining part of its length. The pre-concentrator is controlled by the control module S using the measurement triggering module W. Alternative pre-concentrator solutions available on the market are discussed in detail in the doctoral dissertation: Artur Rydosz, Detekcja gazów o malych koncentracjach z uzyciem mikroprekoncentratorów (Gas detection at low concentration using micropre-concentrators), AGH 2014, in Chapter 2: Gas sample concentrating systems.

Use of the three-way valve Z3 improves precision and repeatability of biomarker concentration detection, as only the end fraction of air exhaled by the patient saturated with biomarkers is pre-concentrated, and concentration of biomarkers remains relatively constant at the end of the exhalation. This facilitates restoration of the initial biomarker concentration, before concentration in the pre-concentrator, on the basis of the measurement with the pre-concentrator.

Signal connections are marked in Fig. 1 with a single line and transmission direction - using arrows. These connections may be provided as wired, optical or wireless radio connections. The control unit S is connected with a carbon dioxide CO2 sensor, from which it receives the measurement signal and, via the measurement triggering module W, with the three way valve Z3, to which it sends the control signal, and with the sensor unit M sending biomarker indications. These connections may be provided using a dedicated I/O system, not presented in the figure. The control module S also communicates with the interface system I. This system includes buttons, wired and/or wireless communication modules, displays and other elements enabling user interaction or integration with other devices, in particular communication with a remote telemedical server. The measurement triggering module W may be provided as an electronic system adapted to generating a valve control signal and a pre-concentrator control signal. The control module S may be alternatively provided as a microcontroller or a FPGA array with adequate converters and the triggering module may be provided as a software element.

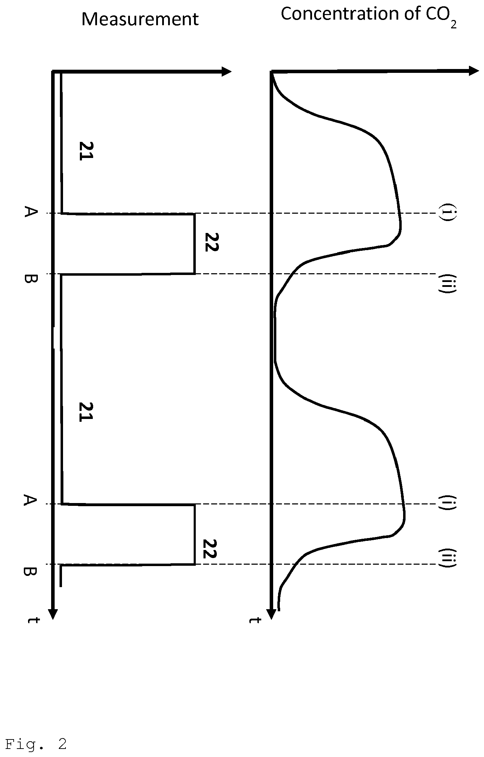

The device according to the invention enables air analysis to be performed along with changes to concentration of exhaled carbon dioxide, such that analysis of biomarker presence includes useful air exhaled at the end of the breath, richest in CO2 and in biomarkers. Thus, CO2 concentration is used as the reference signal, allowing the measurement to be triggered during the appropriate phase of breath. The moment when the threshold concentration is exceeded is used as the criterion in this case. An example profile of CO2 concentration variations and a correlated profile of biomarker detection are presented in Fig. 2. The best conditions for biomarker detection during a breath cycle are indicated as 22 on the bottom graph. The wave indicated as 21 includes suboptimal conditions, under which no measurement is performed according to the invention. Limits (i) and (ii) of phases 21 and 22 are obtained analysing carbon dioxide concentration in the exhaled air - the top graph, which is much higher than biomarker concentrations and easily measured.

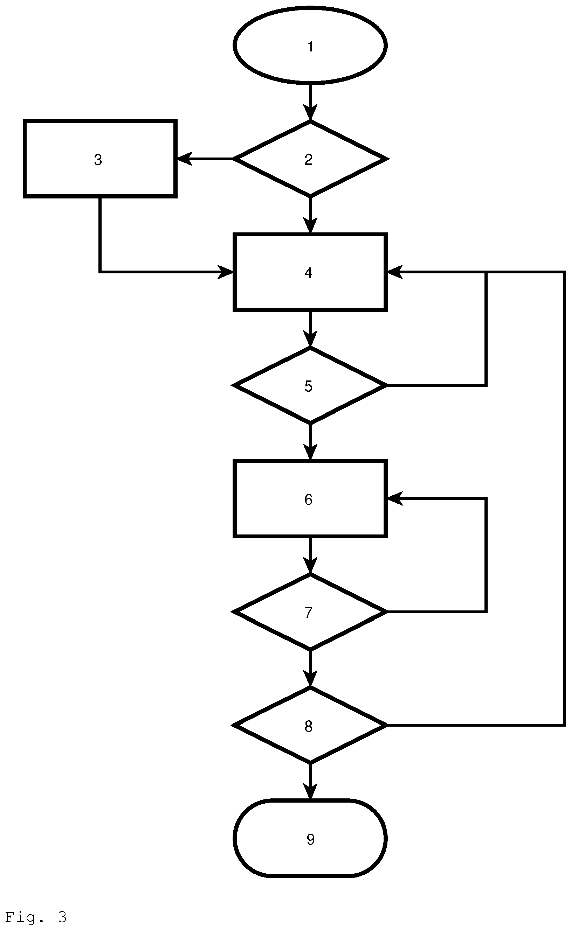

The method according to the invention is executed analysing air blown by the patient into the device through the mouthpiece U placed in the mouth. The sensor unit of the device includes at least one sensor of at least one biomarker, such as acetone. The analysis may be concluded with a quantitative determination - of biomarker concentration values or with a binary value indicating whether the threshold concentration was detected or not. A flow diagram of an example method according to the invention is presented in Fig. 3. During stage 1, when the measurement is initiated, the three way valve is set in position A; this valve is then switched to position B if the indication of the carbon dioxide CO2 sensor exceeds the specified threshold value. Stage 2 includes determination whether a threshold value has been set. This value may have been stored from the previous measurement in the memory of the control unit S or may be input in advance to the device according to the invention using the interface system I.

The threshold value may also be determined using tables, according to patient data, such as e.g. age, weight or on the basis of a training measurement - calibration.

If the threshold value is not determined otherwise, calibration is performed during the calibration stage, for the given patient.

Calibration may be performed, for example, analysing indication of the carbon dioxide CO2 sensor during a cycle of one or more exhalations and by determining the threshold value as a specific fraction of the maximum value. The specified fraction should be greater than 80%. Good efficiency of acetone determination as a biomarker was achieved in the range of 90% - 95%. For most of the population the most appropriate trigger value falls within a range of 91%-93% of maximal concentration.

Then, the actual measurement begins. During the actual measurement, stage 4 of carbon dioxide measurement, the carbon dioxide CO2 concentration is measured and followed by stage 5, in which it is determined whether the threshold value has been exceeded. If carbon dioxide concentration measured using the carbon dioxide CO2 concentration sensor exceeds the specified threshold value, the three way valve Z3 is switched to position B and during this stage, air is directed to the sensor unit M until the end of the exhalation, while stage 6 of biomarker detection includes triggering air release from the pre-concentrator and concentration measurement for at least one biomarker. Air exhaled at the end of the breath cycle - the so called useful air is the best material for biomarker analysis because of the higher biomarker concentrations and because of the fact that such performed measurements are much more repeatable. Concentration measurement performed for at least one biomarker may be quantitative or binary: detected/not detected.

The end of the breath is tested in stage 7 of breath end detection, which may be performed using a flow meter (if the device is provided with a flowmeter) or by detecting a drop in CO2 concentration indicated by the carbon dioxide CO2 concentration sensor. The method according to the invention may be performed by a human or using a software run on the device according to the invention or remotely controlling the device.

Good results were also obtained with fixed measurement time within a range of 1.5 s to 2 s. Process of exhaling air in a function of carbon dioxide concentration is relatively repeatable. End of exhalation is close to local maximum of exhaled CO2 concentration in the breath cycle. Having reached 85% - 90% this concentration is usually stable for some time and then slightly increases to 91-93%, and finally drops. Rapid decrease of CO2 concentration corresponds to the end of breath. Thereafter only small amount of air is exhaled.

Biomarker concentration may be determined more precisely, if measurement is performed over several exhalation, by repeating stage 4 of CO2 concentration measurement with the three way valve set in position A. The test ends if stop criterion is met as determined during stage 8 of stop condition testing. The criterion may include the number of breaths, test time or the signal generated by the patient and recorded using the interface I.

The moisture filter F and the pre-concentrator P can are be integrated within a single unit. This has an advantage as the heater of the pre-concentrator can be used to drying moisture filter F. Heating of the filter surprisingly increases it expected life span.

An example, applicable sensor unit M is provided as the integrated gas sensor array known from the disclosure of the Polish patent application P.417038 (the patent was granted on 29.04.2018). It is a monolithic array made using low temperature, co-fired ceramics LTCC, containing many ceramic layers. One of the layers includes separated arms, comprising thermal bridges with gas sensor heaters located on one side thereof. Through openings feeding gas to the sensors are provided at the layer between gas sensors and the environment, and an air compartment is provided in another layer. A list of other useful sensor types may be found in the following doctoral dissertation: Artur Rydosz, Detekcja gazów o malych koncentracjach z uzyciem mikroprekoncentratorów (Gas detection at low concentration using micropre-concentrators), AGH 2014 - p. 25. Application of such matrix in the present invention requires a branch in the airline to guarantee that not useful air is not going to be fed to pre-condenser.

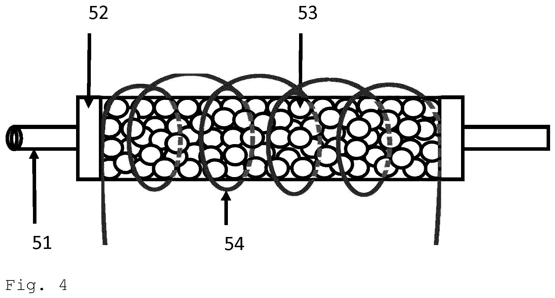

The pre-concentrator P integrated with a moisture filter F is provided according to the invention as a device including an air duct filled with absorbing and adsorbing compounds. Thanks to this solution, the pre-concentrator adsorbs moisture and acts as a water vapour filter. This is advantageous in that the filtration does not reduce concentrations of analysed biomarkers. An example design of the pre-concentrator is shown schematically in Fig. 4. Gas inlet 51 continues as a quartz pipe 52 provided with polymeric adsorbing agent 52 on its walls capturing acetone molecules and with a Ni-CR heater 54. Acetone is adsorbed during exhalation and then released in a shorter time and at a higher concentration, once heated using the heater. Charcoal sieves (not shown) are used to remove acetone. Water is released at a temperature different than temperature used to release acetone. Thus, the same heater may be used to pre-concentrate acetone as a biomarker and to dry charcoal sieves, regenerating the initial condition of the moisture filter. This increases the filter lifetime. The pre-concentrator may also be adapted to biomarkers other than acetone.

Depending on the application, the adsorbing agent may include a composite of various compounds, e.g. organic compounds such as TENAX-TA and compounds based on charcoal sieves CARBOXEN-1018, CARBOXEN-1000 or CARBOSIEVE-SIII. It is also possible to fill the pre-concentrator duct using spherical particles of various types, ensuring removal of water vapour and diabetes biomarkers. Particles with compounds responsible for water vapour adsorption are placed in the initial stage of pre-concentrator filling, such that water vapour is removed at the beginning of the process, and this part is known as the moisture filter or an RH filter. The RH filter should cover 10-30% of the device length, preferably 18-22%. The remaining volume is filled with particles containing and adsorbing agent, e.g. CARBOXEN-1018 offered by Sigma-Aldrich. Higher adsorption efficiency and shorter pre-concentrators may be achieved using a composite of at least two adsorbents TENAX-TA/CARBOXEN-1018.

Measurement interferences caused by adsorbed water vapour may be prevented by specifying a heating profile such that biomarkers are released first, at temp. lower than e.g. 220° C and after some detection time - water vapour is released at 350°-500°C, depending on materials used.

The embodiments of the invention discussed above are intended to explain the invention in a manner enabling its re-creation to people skilled in the art. People skilled in the art, who have read the disclosure, will be easily able to indicate other sensor sand units which may be used, as well as other solutions distributing air within the device. Such people may also change the order of elements, use a bifurcated air line or various working compartments. The method according to the invention may be executed by a person performing the measurement using the central unit or by a person remotely communicating with the device or with the patient. This method may also be executed using a piece of software installed in the device or on a remote server. The control module (S) and the measurement triggering module (W) may be implemented as various solutions - as analogue or digital systems, including processors, microcontrollers or FPGA arrays. They may be implemented in the same or in two different hardware elements. All variations and modifications discussed in this paragraphs are covered by the scope of the claims presented below, which determine the actual scope of protection, otherwise not limited to examples, variants and embodiments discussed in the disclosure.

## Claims

1. A portable device for detection of biomarkers in exhaled air, provided with a control module (S), interface system (I), a mouthpiece (U), a sensor unit (M) and an air line, leading the air from mouthpiece (U) through a sensor unit (M) to an outlet port (O1), wherein the air line includes a moisture filter (F) and the sensor unit (M) is configured for detection of biomarkers and is connected with a control module (S) connected to an interface system (I), wherein
the air line also includes a pre-concentrator (P) and a carbon dioxide sensor (CO2) connected with the control module (S), which is provided with a measurement triggering module (W) configured to trigger a measurement executed with the sensor unit (M) when the carbon dioxide sensor (CO₂) indication exceeds a threshold, the carbon dioxide sensor (CO2) is located in the air line upstream of the sensor unit (M) the device further has a second outlet port (O2) and a three-way valve (Z3) located in the air line downstream of the carbon dioxide sensor (CO2), directing air
to the second outlet port (O2) in its first position (A), a controlled valve cutting off air flow to the sensor unit, and
to the pre-concentrator (P), the sensor unit (M) and the first outlet port (O1) in its second position (B),
wherein the pre-concentrator (P) is integrated with the moisture filter (F) in a single module including an air duct filled with absorbing and adsorbing compounds.

2. The portable device for monitoring of exhaled air composition according to any of claims 1, **characterised in that** the sensor unit (M) includes an acetone sensor.

3. The portable device according to claim 2, **characterised in that** the acetone sensor is a semi-conductor sensor.

4. A method of biomarker detection in exhaled air using the portable device of any one of the previous claims with a sensor unit (M) configured to detect biomarkers, in which an exhaled air stream is fed from the mouthpiece (U) to the outlet (O1) using the air line including the moisture filter (F) and the sensor unit (M), **characterised in that** the exhaled air is subjected to a carbon dioxide concentration measurement using the carbon dioxide sensor (CO2), while biomarker detection begins when the carbon dioxide sensor (CO2) indication exceeds a threshold value, as air flow to the sensor unit (M) is cut off before the analysis using the valve (Z3) and opened again only when the sensor (CO2) indication exceeds the threshold value, then air is concentrated using the pre-concentrator (P) integrated with the moisture filter (F) and then released to enable detection.

5. The method according to claim 4, **characterised in that** before biomarkers are detected using the sensor unit (M), a calibration measurement is performed using the carbon dioxide sensor (CO2), for at least one breathing cycle, and used to determine the threshold carbon dioxide concentration.

6. The method according to claim 5, **characterised in that** the threshold concentration is determined as a fraction in the range of 0.8 to 0.95 of the maximum measured carbon dioxide concentration.

7. The method according to claim 6, **characterized in that** the threshold concentration is selected within a range of 0.91 to 0.93 of the maximal concentration.

8. The method according to claim 4 or 5 or 6, **characterised in that** the threshold value is determined in a measurement covering at least five breathing cycles.

9. The method according to claim 8, **characterised in that** exhaled air is fed to the second outlet port (O2) before the carbon dioxide sensor (CO2) indication exceeds the threshold value.

## Patentansprüche

1. Tragbare Vorrichtung zur Detektion von Biomarkern in ausgeatmeter Luft, die mit einem Steuermodul (S), einem Schnittstellensystem (I), einem Mundstück (U), einer Sensoreinheit (M) und einer Luftleitung versehen ist, die die Luft vom Mundstück (U) durch eine Sensoreinheit (M) zu einer Auslassöffnung (O1) führt, wobei die Luftleitung einen Feuchtigkeitsfilter (F) enthält und die Sensoreinheit (M) zur Detektion von Biomarkern konfiguriert und mit einem Steuermodul (S) verbunden ist, das an ein Schnittstellensystem (I) angeschlossen ist, wobei die Luftleitung auch einen Vorkonzentrator (P) und einen Kohlendioxid-Sensor (CO2) umfasst, der mit dem Steuermodul (S) verbunden ist, das mit einem Messauslösemodul (W) versehen ist, das so konfiguriert ist, dass es eine mit der Sensoreinheit (M) ausgeführte Messung auslöst, wenn die Kohlendioxid-Sensor (CO)-Anzeige einen Schwellenwert überschreitet, der Kohlendioxid-Sensor (CO2) in der Luftleitung stromaufwärts der Sensoreinheit (M) angeordnet ist, die Vorrichtung ferner eine zweite Auslassöffnung (O2) und ein Dreiwegeventil (Z3) aufweist, das in der Luftleitung stromabwärts des Kohlendioxid-Sensors (CO2) angeordnet ist und Luft in seiner ersten Position (A) zur zweiten Auslassöffnung (O2) leitet, ein gesteuertes Ventil, das den Luftstrom zu der Sensoreinheit und zu dem Vorkonzentrator (P), der Sensoreinheit (M) und der ersten Auslassöffnung (O1) in seiner zweiten Position (B) unterbricht, wobei der Vorkonzentrator (P) mit dem Feuchtigkeitsfilter (F) in einem einzigen Modul integriert ist, das einen mit absorbierenden und adsorbierenden Verbindungen gefüllten Luftkanal enthält.

2. Tragbare Vorrichtung zur Überwachung der Zusammensetzung der ausgeatmeten Luft nach einem der Ansprüche 1, **dadurch gekennzeichnet, dass** die Sensoreinheit (M) einen Aceton-Sensor enthält.

3. Tragbare Vorrichtung nach Anspruch 2, **dadurch gekennzeichnet, dass** der Aceton-Sensor ein Halbleiter-Sensor ist.

4. Verfahren zur Detektion von Biomarkern in ausgeatmeter Luft unter Verwendung der tragbaren Vorrichtung eines der vorhergehenden Ansprüche mit einer Sensoreinheit (M), die zur Detektion von Biomarkern konfiguriert ist, bei dem ein ausgeatmeter Luftstrom von dem Mundstück (U) zu dem Auslass (O1) unter Verwendung der Luftleitung, die den Feuchtigkeitsfilter (F) und die Sensoreinheit (M) enthält, zugeführt wird, **dadurch gekennzeichnet, dass** die ausgeatmete Luft einer Kohlendioxidkonzentrationsmessung unter Verwendung des Kohlendioxid-Sensors (CO2) unterzogen wird, während die Biomarker-Detektion beginnt, wenn die Kohlendioxid-Sensor (CO2)-Anzeige einen Schwellenwert überschreitet, da der Luftstrom zu der Sensoreinheit (M) vor der Analyse mit dem Ventil (Z3) unterbrochen und erst wieder geöffnet wird, wenn die Sensor (CO2)-Anzeige den Schwellenwert überschreitet, und dann die Luft mit dem in den Feuchtigkeitsfilter (F) integrierten Vorkonzentrator (P) konzentriert wird und dann freigegeben wird, um die Detektion zu ermöglichen.

5. Verfahren nach Anspruch 4, **dadurch gekennzeichnet, dass** eine Kalibriermessung mit dem Kohlendioxid-Sensor (CO2) für mindestens einen Atemzyklus durchgeführt und zur Bestimmung der Kohlendioxid-Schwellenkonzentration verwendet wird, bevor unter Verwendung der Sensoreinheit (M) Biomarker detektiert werden.

6. Verfahren nach Anspruch 5, **dadurch gekennzeichnet, dass** die Schwellenkonzentration als ein Bruchteil im Bereich von 0,8 bis 0,95 der maximal gemessenen Kohlendioxidkonzentration bestimmt wird.

7. Das Verfahren nach Anspruch 6, **dadurch gekennzeichnet, dass** die Schwellenwertkonzentration innerhalb eines Bereichs von 0,91 bis 0,93 der maximalen Konzentration ausgewählt wird.

8. Verfahren nach Anspruch 4 oder 5 oder 6, **dadurch gekennzeichnet, dass** der Schwellenwert in einer Messung bestimmt wird, die mindestens fünf Atemzyklen umfasst.

9. Verfahren nach Anspruch 8, **dadurch gekennzeichnet, dass** die ausgeatmete Luft der zweiten Auslassöffnung (O2) zugeführt wird, bevor die Kohlendioxid-Sensor (CO2)-Anzeige den Schwellenwert überschreitet.

## Revendications

1. Dispositif portable pour la détection de biomarqueurs dans de l'air expiré, pourvu d'un module de commande (S), d'un système d'interface (I), d'un embout (U), d'une unité de capteur (M) et d'une conduite d'air, conduisant l'air depuis l'embout (U) à travers une unité de capteur (M) jusqu'à un orifice de sortie (O1), dans lequel la conduite d'air inclut un filtre à humidité (F) et l'unité de capteur (M) est configurée pour la détection de biomarqueurs et est raccordée à un module de commande (S) raccordé à un système d'interface (I), dans lequel
la conduite d'air inclut également un pré-concentrateur (P) et un capteur de dioxyde de carbone (CO2) raccordé au module de commande (S), qui est pourvu d'un module de déclenchement de mesure (W) configuré pour déclencher une mesure exécutée avec l'unité de capteur (M) lorsque l'indication du capteur de dioxyde de carbone (CO2) dépasse un seuil,
le capteur de dioxyde de carbone (CO2) est situé dans la conduite d'air en amont de l'unité de capteur (M),
le dispositif ayant en outre un deuxième orifice de sortie (O2) et un robinet à trois voies (Z3) situé dans la conduite d'air en aval du capteur de dioxyde de carbone (CO2), dirigeant de l'air vers le deuxième orifice de sortie (O2) dans sa première position (A), un robinet commandé coupant le flux d'air vers l'unité de capteur, et vers le pré-concentrateur (P), l'unité de capteur (M) et le premier orifice de sortie (O1) dans sa deuxième position (B),
dans lequel
le pré-concentrateur (P) est intégré au filtre à humidité (F) dans un module unique incluant un conduit d'air rempli de composés absorbants et adsorbants.

2. Dispositif portable pour la surveillance de la composition d'air expiré selon l'une quelconque des revendications 1, **caractérisé en ce que** l'unité de capteur (M) inclut un capteur d'acétone.

3. Dispositif portable selon la revendication 2, **caractérisé en ce que** le capteur d'acétone est un capteur à semi-conducteur.

4. Procédé de détection de biomarqueurs dans de l'air expiré utilisant le dispositif portable selon l'une quelconque des revendications précédentes avec une unité de capteur (M) configurée pour détecter des biomarqueurs, dans lequel un courant d'air expiré est introduit depuis l'embout (U) dans la sortie (O1) à l'aide de la conduite d'air incluant le filtre à humidité (F) et l'unité de capteur (M), **caractérisé en ce que** l'air expiré est soumis à une mesure de concentration en dioxyde de carbone à l'aide du capteur de dioxyde de carbone (CO2), tandis que la détection de biomarqueurs commence lorsque l'indication du capteur de dioxyde de carbone (CO2) dépasse une valeur seuil, au moment où le flux d'air vers l'unité de capteur (M) est coupé avant l'analyse à l'aide du robinet (Z3) et à nouveau ouvert uniquement lorsque l'indication du capteur (CO2) dépasse la valeur seuil, alors l'air est concentré à l'aide du pré-concentrateur (P) intégré au filtre à humidité (F) et ensuite libéré afin de permettre la détection.

5. Procédé selon la revendication 4, **caractérisé en ce qu'**avant que des biomarqueurs soient détectés à l'aide de l'unité de capteur (M), une mesure d'étalonnage est effectuée à l'aide du capteur de dioxyde de carbone (CO2), pendant au moins un cycle de respiration, et utilisée pour déterminer la concentration en dioxyde de carbone seuil.

6. Procédé selon la revendication 5, **caractérisé en ce que** la concentration seuil est déterminée sous la forme d'une fraction comprise dans la gamme de 0,8 à 0,95 de la concentration en dioxyde de carbone mesurée maximale.

7. Procédé selon la revendication 6, **caractérisé en ce que** la concentration seuil est sélectionnée au sein d'une gamme de 0,91 à 0,93 de la concentration maximale.

8. Procédé selon la revendication 4 ou la revendication 5 ou la revendication 6, **caractérisé en ce que** la valeur seuil est déterminée dans une mesure couvrant au moins cinq cycles de respiration.

9. Procédé selon la revendication 8, **caractérisé en ce que** de l'air expiré est introduit dans le deuxième orifice de sortie (O2) avant que l'indication du capteur de dioxyde de carbone (CO2) dépasse la valeur seuil.
